# EUROPEAN PATENT APPLICATION

(11) **EP 1 357 193 A1**
(43) Date of publication of application: **29.10.2003**
(21) Application number: 02076573.1
(22) Date of filing: 22.04.2002
(51) Int. Cl.: C12P 17/12, C07D 241/06, A23L 1/23

(54) **Preparation of pyrazines**

(71) Applicant: SOCIETE DES PRODUITS NESTLE S.A., 1800 Vevey (CH)
(72) Inventor: Kurniadi, Toshinari, 1510 Moudon (CH); Belrhlid, Rachid, 1066 Epalinges (CH); Berger, Ralf Günter, 30455 Hannover (DE); Juillerat, Marcel Alexandre, 1000 Lausanne 26 (CH); Fay, Laurent, Bernard, 74500 Evian (FR)
(74) Representative: Wavre, Claude-Alain

(57) **Abstract**

The present invention relates to a new process for the generation of pyrazines consisting in the bioconversion of hydroxyketones with 1,2-diaminopropane. New tetrahydropyrazine derivatives as well as new dihydropyrazine derivatives with high flavour and low threshold properties are disclosed. Such pyrazines compounds exhibiting roasted and earthy aroma profiles may be used in the food and bevererage industry, especially chocolate, confectionery and coffee.

## Description

The present invention relates to the preparation of pyrazines compounds and derivatives thereof as well as compositions containing the same.

Pyrazines are heterocyclic, nitrogen containing compounds which contribute significantly to the flavour of many foods.
Pyrazine compositions and derivatives thereof are known to have utility in varieties of applications is various industries as broad-ranging as the pharmaceutical, tobacco, dyestuff and of course food industries.
Pyrazines derivatives have been known to impart a regular coffee flavor generally described as green by experts as evidenced in the British patent 1 156 475.
US 3622346 discloses the unique nutty, roasted flavors of the methoxypyrazines having potential applications as additives to a variety of baked or toasted foodstuffs.
A row of patents involving the use of pyrazines as flavour enhancers in foods indicate their potential as flavouring agents. Such patents include the addition of pyrazines for popcorn-like, nut-like, coffee-like, pineapple-like and chocolate-like flavours (US 3402051, US 3619210, US 3803331 and GB 1248380).
A plurality of reactants compounds, compositions, reactions mecanisms and conditions have been proposed and suggested in the art for the manufacture of pyrazines and derivatives.
US 3067199 describes the reaction of alkanolamine with nickel or cobalt hydrogenation/dehydrogenation catalysts at elevated temperature under subatmospheric pressure.

In US 3676442, acetylene compounds are reacted with an ammoniacal derivative under acid conditions at elevated pressure.
Japanese Kokai application 52-97983 discloses a process where *hydroxyketone* compound and an ammonium salt of an acid are heated with a heat-stable solvent under neutral or acidic conditions.
As indicated by Rizzi (J. Agric. Food Chem. Vol. 36 N°2, 1988, pp 349-352) who proceed to study the nature of pyrazine formation from *hydroxyketone* compounds and ammonium salts by reaction under reflux conditions, although alkyl pyrazines have been widely investigated as trace components in foods, their exact origin remains unclear. Particularly, Rizzi noted that alkyl pyrazines have been found in fermented products and it is also believe that they are formed during baking, roasting, cooking of various foodstuffs and have consequently great importance as flavor components of such foodstuffs. In particular, it is believed that these compounds impart roast flavor and aromatic characteristic to foodstuffs. Thus efforts are being made to attempt to prepare such compositions in a food acceptable manner.

As noted from all the above mentionned prior art, the procedures to manufacture and isolate pyrazines compounds are generally complex, cumbersome, involve catalysts, solvents, high temperature and/or high pressure conditions, heavy equipement for performing the reaction ...

It thus appears a need for a simple, cheap, direct and food-grade process for the generation of pyrazines compounds.

Accordingly, the present invention provides a process for the preparation of pyrazine derivatives compounds comprising the steps of :
- performing the bioconversion of an aldehyde of the general formula :

   R1-COH

   with a microorganism providing a pyruvate decarboxylase activity in the presence of pyruvate or oxobutyric acid into corresponding hydroxyketone of the general formula :

   R1-CHOH-CO-R2
- and reacting at ambient conditions the obtained hydroxyketone formed with 1,2-diaminopropane, in order to obtain pyrazine derivative compounds,
where R1 is a C1 to C7 alkenyl residue and
R2 is a methyl residue when pyruvate is used or an ethyl residue when oxobutyric acid is used.

R1 may also be a C1 to C7 alkyl residue, and in this case, the reaction of the hydroxyketone with propanediamine is performed after a step involving the concentration of the saturated hydroxyketone.

The expression "ambient conditions" refers to ambient temperature and pression conditions, namelly a temperature ranging between about 15°C and 25°C, and a pression of about the atmospheric pressure, about 950 to 1050 hPa, for exemple.

It is also an object of the present invention to provide new tetrahydropyrazine derivatives of the formula : 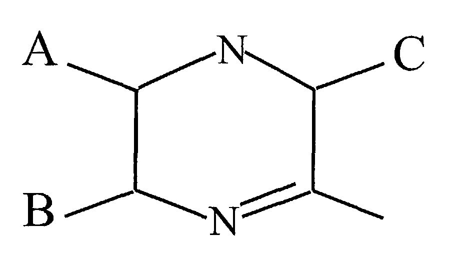 where A and B are identical or different and selected in the group consisting of CH3 and H, C is a C1 to C6 alkyl residue and D is a methyl or an ethyl residue, being unserstood that if C is an ethyl residue D is also an ethyl residue.

It is still an object of the present invention to provide new dihydropyrazine derivatives of the formula : 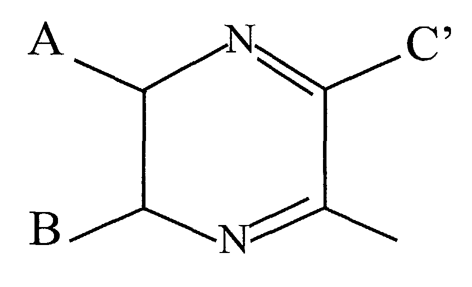 where A and B are identical or different and selected in the group consisting of CH3 and H, C' is a C2 to C7 alkyl residue.

The micro-organism used for the bioconversion of aldehyde into hydroxyketone may be resting cells, disrupted or intact ones. The micro-organism used for the bioconversion may be in the form of an extract, a powder or a cream solution. Preferably, the micro-organism is a fresh solution of grown micro-organism, or of up 4 to 8 days, kept in the refrigerator.
In one preferred embodiment, the micro-organism is selected from the group comprising bacteria, yeasts, moulds and fungus. The preferred bacteria are selected from the group comprising Zymomonas, Lactobacillus and Bacillus. The preferred moulds and fungus are selected from the group comprising Neurosporae and Aspergillae. The preferred yeasts are selected from the group comprising Saccharomyces, Debaromyces, Candida, Pichia, Schizosaccharomyces and Zygosaccharomyces. More preferably, the yeast is baker's yeast, Saccharomyces cerevisiae.
R1 is preferably an ethylen group and the aldehyde is therefore acrolein that will lead upon bioconversion with pyruvate in the presence of baker's yeast cells to 3-hydroxy-4-pentene-2-one. The bioconverion may also lead to the isomer 2-hydroxy-4-penten-3-one, depending upon reactants relative aboundance. This bioconversion is achieved according to the following route :

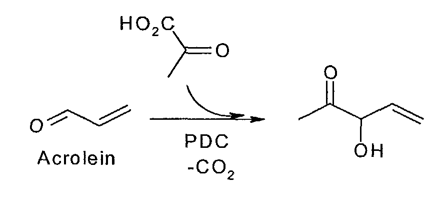

The concentrations of the starting products in the medium, e.g. aldehyde, pyruvate and oxobutyric acid may be in the range of 0.5 mM to 500 mM for aldehade and in the range of from 0.5 to 1000 mM for pyruvate and oxobutyric acid.

Regarding the respective quantities of the starting products aldehyde, pyruvate and oxobutyric acid, they can be such that the molar ratio aldehyde:pyruvate and aldehyde:oxobutyric acid may vary from 1:2000 to 500:1. Optimal ratio aldehyde:pyruvate and aldehyde:oxobutyric acid will depend on the relative aboundance of the desired isomer.
Of course, pyruvate and oxobutyric acid may be used in combination ; this giving a mixture of different hydroxyketones.
In the case of the use of acrolein, the more pyruvate is present in the medium, compared to acrolein, the more 3-hydroxy-4-pentene-2-one is produced and alternatively the less pyruvate is present in the medium compared to acrolein, the more 2-hydroxy-4-pentene-3-one is produced. One has to keep in mind that 3-hydroxy-4-pentene-2-one is however preferentially formed.
The aldehyde may be added in the medium completly or may be advantageously added in the bioconversion medium by mean of fed-batch mode. Such progressive addition of aldehyde may allow to reduce the inhibitory effect of such compound on the formation of hydroxyketones.
Generally the micro-organism solution is used as from 1 to 30% w/v of micro-organism cells in the reaction medium.

The bioconversion may be carried out under aerobic or anaerobic conditions during from 15 minutes to 48 hours, preferably from 1 to 5 hours and at a pH of from 4 to 8, preferably from 5 to 7, more preferably about 6.
The cells of the micro-organism may be immobilized on a solid support such as bead or membrane by appropriate means such as encapsulation for example.
The temperature of the bioconversion may range from 4 to 45°C, preferably from 20 to 35°C.

The reaction medium may be water or buffer such as citrate phosphate or Tris buffers of concentration between 50 to 200 mM, for example. The reaction medium may also comprise ethanol or methanol in a concentration of up to 5% w/v which may serve as increasing the bioconversion yield.

Some co-factors of the decarboxylation like Thiamine pyro phosphate (TPP) and Mg⁺⁺ are generally present in the cells of the micro-organism and therefore in the bioconversion medium. Such compounds may also be added in the medium in order to enhance the bioconversion yield. Mg⁺⁺ may be added in the medium as MgCl₂ or MgSO₄ in order to obtain a concentration of free Mg⁺⁺ in the range of from 0.1 mM to 20 mM. TPP may be added in them medium in the range of from 0.1 mM to 10 mM.

The pyrazine derivatives refered to in the present application may be selected in the group comprising the following pyrazines derivatives compounds :
2,3,5-trimethyl-5,6-dihydropyrazine
2-ethyl-3,6-dimethyl-5,6-dihydropyrazine
2-ethyl-3,5-dimethyl-5,6-dihydropyrazine
2,3-diethyl-5-methyl-5,6-dihydropyrazine
2-propyl-3,6-dlmethyl-5,6-dihydropyrazine
2-propyl-3,5-dimethyl-5,6-dihydropyrazine
2-pentyl-3,6-dimethyl-5,6-dihydropyrazine
2-pentyl-3,6-dimethyl-5,6-dihydropyrazine
2-hexyl-3,6-dimethyl-5,6-dihydropyrazine
2-hexyl-3,6-dimethyl-5,6-dihydropyrazine
2-heptyl-3,6-dimethyl-5,6-dihydropyrazine
2-heptyl-3,5-dimethyl-5,6-dihydropyrazine
2,3,5-trimethyl-1,2,5,6-tetrahydropyrazine
2-ethyl-3,6-dimethyl-1,2,5,6-tetrahydropyrazine
2-ethyl-3,6-dimethyl-1,2,5,6-tetrahydropyrazine
2-propyl-3,6-dimethyl-1,2,5,6-tetrahydropyrazine
2,3-diethyl-5-methyl-1,2,5,6-tetrahydropyrazine
2-propyl-3,5-dimethyl-1,2,5,6-tetrahydropyrazine

2-ethyi-3,6-dimethylpyrazine
2-ethyl-3,5-dimethylpyrazine
2,3-diethyl-5-methylpyrazine
2-pentyl-3,6-dimethylpyrazine
2-pentyl-3,5-dimethylpyrazine
2-heptyl-3,6-dimethylpyrazine
2-heptyl-3,5-dimethylpyrazine

Indeed, it is the very first time that tetrahydropyrazine compounds are found to be flavour active. Regarding the dihydropyrazine compound according to the present invention, the odour threshold as well as flavour profile are respectivelly much lower and more equilibrated as compared to the existing ones. For instance, the odour threshold of 2-ethyl-3,5-dimethyl-dihydropyrazine is at least 1000 time less than the odour threshold of 2,3,5-dimethyl-5,5-dihydropyrazine.

The reaction of the obtained hydroxyketone with 1,2-diaminopropane (DAP) in order to produce pyrazine derivatives may be achieved in a medium such as the bioconversion medium used for the generation of hydroxyketone. Therefore, 1,2 diaminopropane may be added to the bioconversion medium containing hydroxyketone in a ratio of 1:1 to 1:2 hydroxyketone:1,2 diaminopropane. However, DAP may aldo be added to and reacted with purified hydroxyketone in an aqueous based medium but also in an other non aqueous medium like ether, ethanol or methanol for exemple. In such case the hydroxyketone are extracted from the bioconversion medium and dissolved in the aqueous or non-aqueous medium and eventually the DAP is added and the reaction performed at room temperature as previously exposed.

It is thus an other aspect of the present invention to provide a process for the generation of pyrazine derivatives consisting in :
- reacting at least one hydroxyketone according to the formula :

   R1-CHOH-CO-R2

   with 1,2-diaminopropane at ambient condition, in order to obtain pyrazine derivatives compounds,
where R1 is a C1 to C7 alkenyl residue and
R2 is a methyl or an ethyl residue.

The reaction of hydroxyketone with DAP may be done at room temperature, such as from 18 to 25°C for exemple, but it may be performed at higher temperatures in order to accelerate the reaction. Indeed it is the first time that generation of pyrazines compounds is obtained by a combination of biotransformation and chemical reaction at such low temperatures. The reaction may be carried out at such temperature during a time sufficient to generate the pyrazines derivatives, for instance during from about 1 hour to about 20 hours depending on the temperature used. The aqueous phase containing the pyrazine derivatives may be extracted with diethyl ether for example for characterization by means of gas chromatography. The extraction procedure may be repeated twice or three times.

All in the whole, various pyrazines, most dihydropyrazines and all tetrahydropyrazines produced according to the present application are novel. Some of the novel pyrazine derivatives were shown to possess pleasant roasted or earthy aroma properties and low odour thresholds. Especially the tetrahydropyrazine derivatives are interesting candidates because nothing has been described in literature in terms of flavour properties.

New roasted or earthy aroma properties are for example interesting for beverage industry as well as chocolate and confectionery one. Accordingly it is also an object of the present invention to use of the pyrazines derivatives according to the present invention in food products, especially beverages for purpose of taste and flavour improvement.

### Materials

Dried baker's yeast for bioconversion was purchased by Hefe Schweiz. All chemicals were from Sigma Aldrich Chemical Co.

### Methods

### Biotransformation using Baker's Yeast for hydroxyketones production

Biotransformation studies were carried out with commercially-available dried baker's yeast in shaking flasks. To a solution of Baker's Yeast in 0.1 M sodium citrate buffer (2 mM thiamine pyrophosphate, 20 mM magnesium sulfate, pH 6.0), 2-oxocarboxylic acid and aldehyde in different concentrations were simultaneously added. A kinetic study was performed by collecting samples, centrifugating them and further analytical treatment of the supernatant.

### Biotransformation using Baker's Yeast for the production of pyrazine derivatives

Biotransformation studies were carried out with commercially-available dried baker's yeast in shaking flasks at room temperature. 25 mM 2-oxocarboxylic acid and 25 mM aldehyde were subjected to biotransformation using a 4 % suspension of Baker's Yeast in 0.1 M sodium citrate buffer (2 mM thiamine pyrophosphate, 20 mM magnesium sulfate, pH=6.0), and 60 minutes later 10 times excess of 1,2-diaminopropane (1,2-DAP) was added. After a total incubation of 150 minutes, analysis of volatiles was performed by gas chromatography.

### Quantitation of hydroxyketones

After centrifugation of collected samples, 4-hydroxy-4-methyl-2-pentanone was added as internal standard. The aqueous phase was extracted twice with one volume of distilled diethyl ether. The ether extract was dried over anhydrous Na₂SO₄ and concentrated using a Vigreux column at 40 °C. Quantitation was performed by comparing the surfaces of peaks between the concerned products and the internal standard in the GC chromatogram. Due to structural similarity of products and internal standard the FID response factors of both are suggested to be equal.

Gas Chromatography (GC) and Gas Chromatography / Olfactometry (GC-O)

GC-analyses were performed on a Agilent 6890 Series GC equipped with a Splitless injector, a flame ionization detector (FID) and sniffing port. Separation of volatiles was either achieved on a DB-Wax capillary column or a DB-1 capillary column (30 m x 0.25 mm, film thickness 0.25 mm, J & W Scientific) using helium as carrier gas (1.5 mL min⁻¹) and nitrogen (45 kPa) as make up-gas for the FID. The following temperature programs were applied:
With DB-Wax:
   5 min isothermal at 40 °C, then raised to 220 °C at 5 °C/min and kept 5 min isothermal at 220 °C.
With DB-1:
   5 min isothermal at 40 °C, then raised to 240 °C at 4 °C/min and kept 15 min isothermal at 240 °C.

### Gas Chromatography/ Mass spectometry (GC/MS)

GC-MS analyses were performed on a Finnigan MAT-8430 mass spectrometer combined with an HP 5890 gas chromatograph using the same GC conditions as described above. The MS-EI spectra were generated at 70 eV and MS-CI at 150 eV with ammonia as reagent gas.

### Extraction procedure of 3-hydroxy-4-penten-2-one

After removal of cells by centrifugation, the aqueous supernatant (400ml) was extracted continuously with 800 mL of distilled diethyl ether overnight. The extract was dried over anhydrous Na₂SO₄ and concentrated using a Vigreux column at 40 °C.

### Purification procedure of 3-hydroxy-4-penten-2-one

3-Hydroxy-4-penten-2-one was purified by High Performance Liquid Chromatography (HPLC) using a Hewlett Packard series 1050 device, equipped with a diode array detector. Separation of compounds was achieved by using a normal phase column (Nucleosil 100-7-OH, 4x250 mm, Macherey Nagel). Elution was carried out using pentane/diethylether at a flow rate of 1.0 ml/min. The concentration of diethylether in the eluent was gradually increased from 0 % to 30 % during the first 30 minutes. UV-detection was done at 210 nm and 3-hydroxy-4-penten-2-one was repetitively collected between 21 and 23 min.

### Results and discussion

### Production of 3-hydroxy-4-pentene-2-one (3-HPO) by biotransformation using Baker's Yeast

With whole cells 3-HPO was formed during the first four hours of incubation and higher substrate concentration were favourable for formation of 3-HPO in a range from 10 mM to 50 mM. Best product concentrations (150 mg/l) were obtained using a 20% suspension of Baker's Yeast, substrate concentrations of 50 mM and incubation for 3 hours. 3-HPO was purified by HPLC.

Biotransformation led also to the production of 2-hydroxy-4-penten-3-one (2-HPO), the isomer of 3-HPO. The quantitative ratio between 3-HPO and 2-HPO was dependent on the relative amounts of aldehyde and pyruvate. When equimolar concentrations of pyruvate and aldehyde were used, 3-HPO was produced in excess. When using pyruvate in excess, even more 3-HPO was produced relatively to 2-HPO than in case of equimolarity whereas the opposite relationship was observed with higher aldehyde concentrations. However, amounts of 2-HPO have never exceeded those of 3-HPO. It was found that the hydroxyketones were even produced when only the aldehyde was subjected to biotransformation without addition of pyruvate to the medium. This is due to the fact that endogeneous pyruvate is always present in the cell. Under this condition approximately same amounts of 3-HPO and 2-HPO were produced.

Furthermore, 4-pentene-2,3-dione and γ-pentalactone were identified as product of biotransformation.

### Production of a pool of hydroxyketones using S.cerevisiae

In analogy, pyruvate and other linear aldehydes were subjected to biotransformation using S. *cerevisiae*. In a special case acrolein and 2-oxo butyric acid were taken as substrates. After one hour of incubation at 25 mM initial substrate concentrations numerous hydroxyketones were identified. The product yields after one hour of incubation were strongly dependent on the choice of aldehyde, 2-hexanal being the best candidate with yields above 50 % in terms of the 3-hydroxy isomer, for example. In general it was observed that branched aldehydes were converted worse than linear ones, unsaturated aldehydes worse than saturated ones and short aldehydes worse than long ones.
In conclusion a pool of hydroxyketones was synthesized by biotransformation of aldehydes using Baker's yeast.

| No. Hydroxyketones | Rl ^{a} | Rl ^{a} Ref | MS Ref |
|---|---|---|---|
| 1 3-hydroxy-2-butanone | 1263 | - | - |
| 2 3-hydroxy-2-pentanone | 1344 | 1346 | Neuser (1999) |
| 3 2-hydroxy-3-pentanone | 1361 | 1346 | Neuser (1999) |
| 4 2-hydroxy-3-hexanone | 1423 | 1434 | Neuser (1999) |
| 5 3-hydroxy-2-hexanone | 1430 | 1440 | Neuser (1999) |
| 6 2-hydroxy-3-heptanone | 1551 | 1539 | Neuser (1999) |
| 7 3-hydroxy-2-heptanone | 1560 | 1547 | Neuser (1999) |
| 8 2-hydroxy-3-octanone | 1647 | 1645 | Neuser (1999) |
| 9 3-hydroxy-2-octanone | 1655 | 1655 | Neuser (1999) |
| 10 2-hydroxy-3-nonanone | 1757 | 1752 | Neuser (1999) |
| 11 3-hydroxy-2-nonanone | 1768 | 1765 | Neuser (1999) |
| 12 2-hydroxy-3-decanone | 1874 | 1859 | Neuser (1999) |
| 13 3-hydroxy-2-decanone | 1889 | 1867 | Neuser (1999) |
| 14 3-hydroxy-5-methyl-2-hexanone | 1479 | 1472 | Neuser (1999) |
| 15 2-hydroxy-4-penten-3-one | 1372 | - | - |
| **16 3-hydroxy-4-penten-2-one** | **1381** | **-** | **-** |
| 17 3-hydroxy-4E-hexen-2-one | 1481 | 1504 | Neuser (1999) |
| 18 2-hydroxy-4E-hexen-3-one | 1511 | 1511 | Neuser (1999) |
| 19 3-hydroxy-4E-octen-2-one | 1683 | 1687 | Neuser (1999) |
| 20 2-hydroxy-4E-octen-3-one | 1698 | 1710 | Neuser (1999) |
| **21 3-hydroxy-4E-nonen-2-one** | **1776** | **-** | **-** |
| **22 2-hydroxy-4E-nonen-3-one** | **1796** | **-** | **-** |
| **23 3-hydroxy-4E-decen-2-one** | **1946** | **-** | **-** |
| **24 2-hydroxy-4E-decen-3-one** | **1959** | - | - |
| 25 4-hydroxy-3-hexanone | 1389 | - | - |
| 26 3-hydroxy-5-hexen-4-one | 1430 | - | - |
| **27 4-hydroxy-5-hexen-3-one** | **1453** | **-** | **-** |

| | | | |
|---|---|---|---|
| ^{a} Linear retention index on a DB-Wax colums | | | |

### α,β-Unsaturated hydroxyketone compounds react with 1,2-DAP to alkyldihydropyrazines

Syntheses with Baker's yeast were performed with other α,β-unsaturated aldehydes, pyruvate and 1,2-DAP. In a particular essay acrolein and 2-oxobutyric acid were used as substrates. RI and GC-MS data strongly suggest that in all reactions alkyldihydropyrazines and not alkenyltetrahydropyrazines were found. Putatively identified 2-propyl-, 2-pentyl-, 2-hexyl- and 2-heptyldimethyl,-5,6-dihydropyrazine derivatives contain a prominent mass fragment of 124 while 2-ethyldimethyl-5,6-dihydropyrazines do not. As reference, a mixture of 2-propyldimethyl-5,6-dihydropyrazine isomers as well as 2,3-diethyl-5-methyl-5,6-dihydropyrazine were chemically synthesized.

### Mild synthesis of novel tetrahydropyrazine derivatives

In order to produce tetrahydropyrazines, syntheses with Baker's yeast were performed with saturated aldehydes, pyruvate and 1,2-DAP like those performed before. However, no pyrazine derivatives were obtained although the saturated hydroxyketones were sometimes produced in higher concentrations than the α,β-unsaturated ones before. This result can be explained by the fact that only unsaturated hydroxyketones tautomerize into the highly reactive diene structure while saturated ones do not. In consequence, when reactions between saturated hydroxyketones and diamines were carried out *in vitro* with higher substrate concentrations tetrahydropyrazines could indeed be synthesized.

## Claims

1. Process for the preparation of pyrazine derivatives compounds comprising the steps of :
- performing the bioconversion of an aldehyde of the general formula :
R1-COH
with a microorganism providing a pyruvate decarboxylase activity in the presence of pyruvate or oxobutyric acid into corresponding hydroxyketone of the general formula :
R1-CHOH-CO-R2
- and reacting at ambient conditions the obtained hydroxyketone formed with 1,2-diaminopropane, in order to obtain pyrazine derivatives compounds,
where R1 is a C1 to C7 alkenyl residue and
R2 is a methyl residue when pyruvate is used or an ethyl residue when oxobutyric acid is used.

2. Process according to claim 1, **characterized in that** R1 is a C1 to C7 alkyl residue, and in this case, the reaction of the hydroxyketone with propanediamine is performed after a step involving the concentration of the saturated hydroxyketone.

3. Tetrahydropyrazine derivatives of the formula : 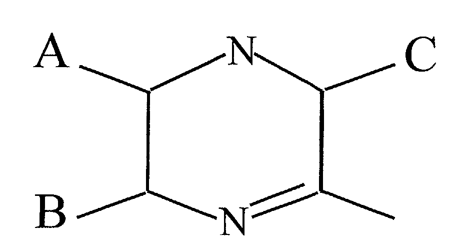 where A and B are identical or different and selected in the group consisting of CH3 and H, C is a C1 to C6 alkyl residue and D is a methyl or an ethyl residue, being unserstood that if C is an ethyl residue D is also an ethyl residue.

4. Dihydropyrazine derivatives of the formula : 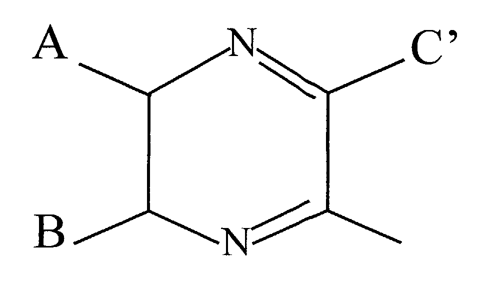 where A and B are identical or different and selected in the group consisting of CH3 and H, C' is a C2 to C7 alkyl residue.

5. Process according to claim 1, **characterized in that** the microorganism is selected from the group comprising Zymomonas, Lactobacillus, Bacillus, Neurosporae, Aspergillae, Saccharomyces, Debaromyces, Candida, Pichia, Schizosaccharomyces and Zygosaccharomyces.

6. Process according to claim 1, **characterized in that** the microorganism is baker's yeast, Saccharomyces cerevisiae.

7. Process for the generation of pyrazine derivatives consisting in :
- reacting at least one hydroxyketone according to the formula :
R1-CHOH-CO-R2
with with 1,2-diaminopropane at ambient condition, in order to obtain pyrazine derivatives compounds,
where R1 is a C1 to C7 alkenyl residue and
R2 is a methyl or an ethyl residue.

8. Use of pyrazines derivatives according to claims 1 to 7 in food products, especially beverages for purpose of taste and flavour improvement.
